# EUROPEAN PATENT APPLICATION

(11) **EP 1 444 955 A1**
(43) Date of publication of application: **11.08.2004**
(21) Application number: 01274707.7
(22) Date of filing: 14.11.2001
(51) Int. Cl.: A61B 10/00

(54) **METHOD AND DEVICE FOR SPECIFYING AFFECTED PARTS**

(71) Applicant: Sakamoto, Noriyasu, Tokyo 179-0076 (JP)
(72) Inventor: Sakamoto, Noriyasu, Tokyo 179-0076 (JP)
(74) Representative: Staudt, Hans-Peter, Dipl.-Ing.
(86) International application number: PCT/JP2001/009944
(87) International publication number: WO 2003/041588

(57) **Abstract**

A method of specifying affected parts characterized by comprising the steps of detecting radiation emitted from a human body in a pinpointed manner by a radiation detector (1), inputting a detected radiation dose or intensity to a computer (3), and displaying radiation doses or intensities at respective portions of the body on a body distribution diagram on a monitor screen (4). The above radiation doses or intensities are measured while the radiation detector (1) is being gradually distanced from the body, and maximum distances at which the radiation detector can detect radiation rays are set as measurements, thereby obtaining data simply.

## Description

### TECHNICAL FIELD

The present invention relates to a method and device for detecting and specifying an area of visceral disorder and an area of neural disorder such as stress by measuring the dose or intensity of radiation emitted from a human body.

### BACKGROUND ART

The applicant applied earlier for a patent for a radiation tester used for the same purpose as the present invention (Japanese Patent Application Laid-Open Publication No. 1994-130152).

The application for a patent is comprised of collecting radiation of millimeters in wavelength from the patient, guiding radiation into a converging chamber via a diaphragm hole, changing radiation into an electrostatic property on a testing plate disposed at the rear portion of the converging chamber, generating a frictional force in the testing plate by the intensity of the radiation and having the friction detected by a skilled technician with a finger tip, thus allowing measurement of the intensity of radiation.

The aforementioned prior art had the problems listed below.

Firstly, the above radiation tester, designed for skilled technicians to detect friction with his or her finger tip, is unable to quantify the dose or intensity of radiation. This makes it difficult, if various areas of a human body are inspected, to record the radiation status at individual areas in a data form, involving difficulties in specifying affected parts.

Secondly, intended to make measurement based on change in friction grasped with a finger tip, the radiation tester can only be handled by skilled persons, possibly resulting in measurement error if the person in charge of measurement is lowly skilled.

Thirdly, all waves collected by the horn are guided to the testing plate via the converging chamber. This has, if electromagnetic waves other than radiation emitted from human body are included, hindered accurate measurement, possibly resulting in lack of measurement reliability.

It is an object of the present invention to solve these technical problems of the prior art.

### DISCLOSURE OF THE INVENTION

The applicant found that, during examination of radiation of a number of patients using the prior art radiation tester, radiation emitted from a human body is detected even far from the human body when the dose or intensity thereof is large and is detected only close to the human body if the dose or intensity thereof is small.

The present invention is based on the knowledge and allows for acquisition of the dose or intensity of radiation, previously difficult to obtain, as quantified numeric data by obtaining the dose or intensity of radiation as numeric data in which the dose or intensity of radiation is replaced with the distance from the human body. However, expressing the dose or intensity of radiation in numerical value by replacing with the distance is not absolutely necessary. It is possible, if a sensor-equipped detector capable of outputting the dose or intensity of radiation as numerical value, to use the dose or intensity of radiation as numerical data by making available an appropriate numerical scale.

The invention according to claim 1 relates to a method of specifying affected parts and is characterized in that radiation emitted from a human body is detected in a pinpointed manner by a radiation detector and the dose or intensity of detected radiation is entered into a computer, thus displaying the dose or intensity of radiation for each area of the human body in a human body distribution diagram on the monitor screen.

Here, the term "in a pinpointed manner" is not limited to pinpointed and extremely small areas and includes those spanning about 10 centimeters in diameter.

The invention according to claim 2 is characterized in that measurement of the dose or intensity of radiation is conducted as the radiation detector is gradually moved farther from the human body and that the maximum distance at which radiation is detected by the radiation detector is used as a measurement.

When the measurement is displayed on the monitor screen, it is preferred that the measurement be divided into a plurality of grades and displayed in a different color in each grade (claim 3).

The invention according to claim 4 relates to a device for obtaining a measurement for carrying out the inventions according to claims 1 to 3 and comprises a radiation detector for detecting in a pinpointed manner radiation emitted from a human body and a distance measurement device for measuring the distance between the human body and the radiation detector.

While the aforementioned conventional radiation tester may be used as the radiation detector, the radiation detector allows detection without experience if configured by a wave collecting unit provided on one side of the casing for collecting waves emitted from a human body in a pinpointed manner, a sensor provided at the rear of the wave collecting unit for detecting radiation and a detection/display unit that remains active while the sensor senses radiation (claim 5).

The sensor is designed such that an audio or optical alarm is given out when the dose emitted exceeds the meter range or a given dose.

The wave collecting unit allows for efficient convergence of collected radiation into the measuring instrument if made in a conical form having an opening large in diameter and tapered toward the rear.

The invention according to claim 6 is a device capable of specifying radiation to be measured by providing, between the wave collecting unit and the sensor, a filter that passes only radiation of a given wavelength.

The filter passes only radiation emitted from the patient. While millimeter wave and far infrared ray are considered to be emitted from affected parts, the present invention includes, if any other radiation is discovered by future research, use of a filter that passes newly found waves.

The invention according to claim 7 is a distance measurement device made into a distance measurement sensor that can be worn in the palm of the hand or a finger. It is further convenient if the distance sensor is integrally configured with the radiation detector.

According to the method of the present invention, it is possible to collect human radiation in a pinpointed manner and obtain the dose or intensity thereof as quantitative numeric data. This data is entered into a computer and displayed in a human body distribution diagram on the monitor screen, thus allowing easy specification of affected parts and commanding an overall bird' s-eye view of the whole body condition for holistic medical care.

According to the device of the present invention, it is possible to collect radiation emitted from a human body in a pinpointed manner and obtain, thanks to use of a sensor for detection, accurate data without experience. Only radiation of a specific wavelength is extracted by a filter, thus cutting out radiation other than that radiated by affected parts and electromagnetic waves other than those radiated by human body during measurement.

According to the present invention, therefore, the above conventional problems will be all solved.

### BRIEF DESCRIPTION OF THE DRAWINGS

Fig. 1 is a schematic view of a device used for carrying out the present invention;
Fig. 2 is a flowchart of an embodiment of the present invention;
Fig. 3 shows an example of human body distribution diagram displayed on the monitor before operation in the present invention;
Fig. 4 shows an example of human body distribution diagram displayed on the monitor after operation in the present invention;
Fig. 5 is a front view of a first embodiment of a radiation detector of the present invention; and
Fig. 6 is a front view of a second embodiment of the radiation detector.

### BEST MODE FOR CARRYING OUT THE INVENTION

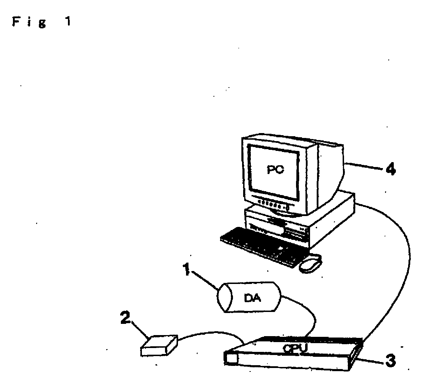

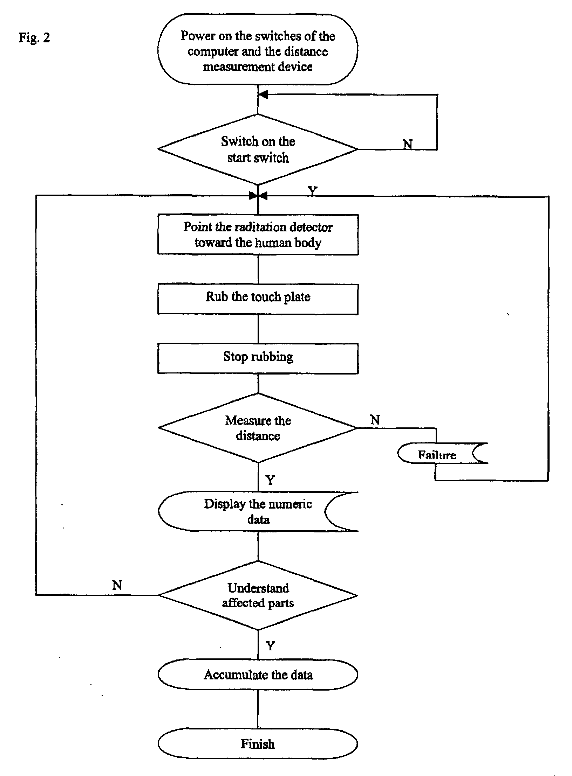

Fig. 1 illustrates a device for carrying out the invention of the affected part specifying method, with symbols 1, 2, 3 and 4 indicating a radiation detector, a distance measurement device that can be worn on the hand or arm, a CPU and a computer monitor, respectively. Fig. 2 illustrates a flow of the embodiment of the present invention.

First, the power switches of the computer 4 and the distance measurement device 2 are switched on, and then the start switches thereof are switched on, thus completing preparations for the measurement.

Next, the radiation detector 1 is pointed toward an area of the human body to be measured, measuring the dose or intensity of radiation. At this time, the radiation detector is first brought close to the human body and then gradually moved away therefrom. The dose of radiation collected by the radiation detector gradually decreases as the detector is moved away, and at a certain distance the radiation detector no longer detects radiation.

If a conventional radiation tester is used, the radiation is detected based on the magnitude of friction resulting from a technician rubbing a touch plate provided on the rear surface of the radiation tester, whereas when a sensor is used, the radiation is detected based on the sensor output.

After the position is found where radiation is undetectable by the function of the radiation detector, the distance from the human body to the position is measured. While this measurement may be made using a rule, it is quicker and more accurate to use the distance sensor.

Next, the distance is entered into the CPU 3. If the distance is measured with a rule, the value is entered from the keyboard. When the distance is measured with the sensor, an electric signal is sent out from the sensor to the CPU.

In the CPU 3, entered distance numeric data is ranked into several grades and displayed in the human body distribution diagram 3 on the monitor 4 as color data associated with each rank.

It is possible to understand affected parts by observing the human body distribution diagram 3 showing the radiation data in different colors.

Then, the disorder is identified in consideration of the locations of affected parts and the symptoms complained of by the patient, administering appropriate treatment.

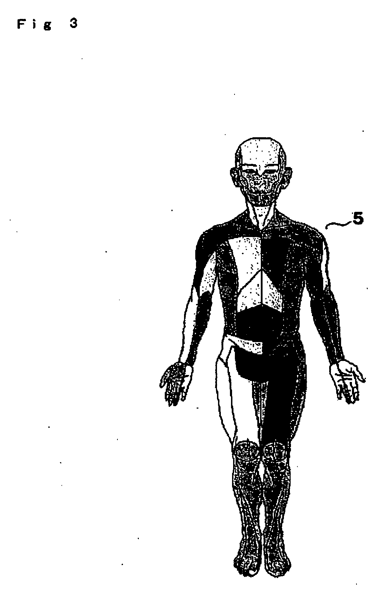

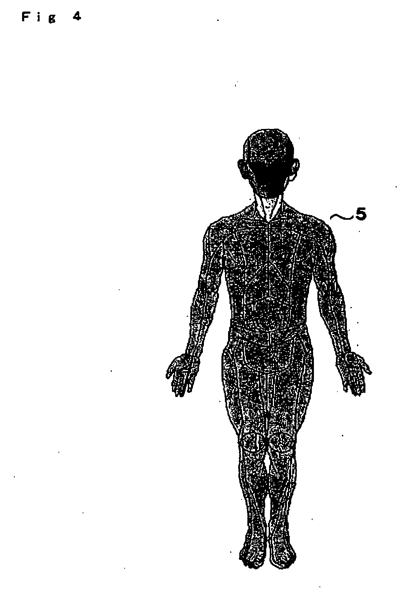

Figs. 3 and 4 show examples of human body distribution diagram before and after operation in which the dose of radiation is evaluated in five different grades, with the radiation-abundant areas indicated by dark colors. While areas with abundant radiation are distributed over the entire human body before the operation, such areas with abundant radiation remain only in specific areas after the operation, making evident the effect of the operation.

Tables 1 and 2 show the relationship between the human body distribution diagram evaluating the dose of radiation in five different grades using the method of the present invention and the areas where pain sensation is perceived by subjects.

Both Table 1 showing the pre-operation condition and Table 2 showing the post-operation condition have confirmed that the grades of the radiation-abundant areas obtained by the method of the present invention match with the areas where pain sensation is perceived for most of the subjects.

That is, the present invention attempting to specify affected parts based on the dose of radiation has been substantiated to be effective.

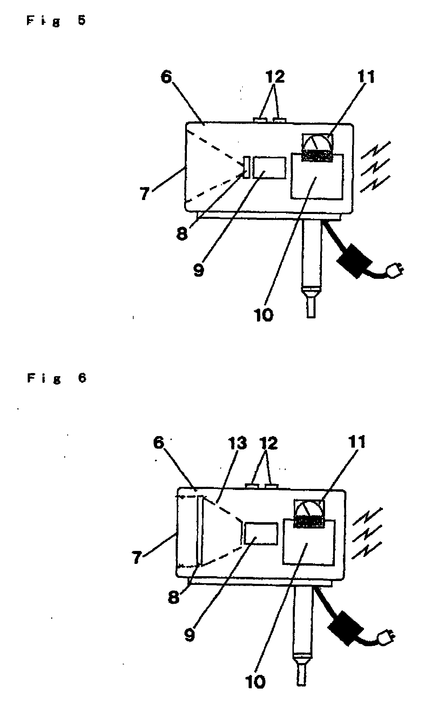

Fig. 5 illustrates an embodiment of the radiation detector of the present invention.

In Fig. 5, there is provided, on one side of a casing 6, a wave collecting unit 7 for collecting, in a pinpointed manner, waves emitted from a human body, whereas there is provided, at the back of the wave collecting unit 7, a filter 8 for passing radiation of a given wavelength, with a sensor 9 provided at the back of the filter 8 for measuring radiation passing through the filter 8.

The wave collecting unit 7 is conical in form having an opening portion 7a large in diameter (e.g., 10 centimeters in diameter) and tapered toward the rear, with the filter 8 in contact with the rear end of the wave collecting unit 7. The filter 8 is designed to pass only far infrared ray.

The sensor 4 is intended to detect the dose and temperature of far infrared ray passing through the filter 8. Since radiation emitted from a human body is targeted for measurement, the sensor 4 is configured such that temperatures from 35 to 42°C can be measured.

The measurement results of the sensor 9 are fed to a control unit 10, showing the temperature data on a meter 11 and indicating the radiation dose with a sound. That is, at areas other than affected parts, radiation is low in temperature and small in dose emitted, thus producing a non-alarming sound. At affected parts, radiation is high in temperature or large in dose emitted, thus producing an alarming sound in the event of a high temperature or large emitted dose.

In the figure, symbol 12 is an ON/OFF switch.

To specify affected parts by the device according to the above embodiment, the switch 12 is switched on and then the opening portion 7a of the wave collecting unit 7 is moved while kept close to the human body. At areas other than affected parts while the opening portion 7a is moved, the control unit 10 produces a non-alarming sound. When an area is reached where radiation is high in temperature or large in dose, the control unit 10 produces an alarming sound.

When an alarming sound is produced, the wave collecting unit 7 is gradually moved away from the human body, reducing radiation at a certain point and changing the sound from an alarming to a non-alarming one. Then, the distance from the human body to where the sound changed is measured. The magnitude of this distance constitutes a numeric value representing the magnitude of dose of radiation.

For measurement of the distance, it would be beneficial to use a distance sensor shaped so as to be held by the palm of the hand for use or install the distance sensor near the opening end of the wave collecting unit 7 of the radiation detector so that the measurement of the radiation sensor can be automatically recorded by the CPU.

According to the above embodiment, the opening portion 7a of the wave collecting unit 7 is conical in form having a large diameter, allowing collection of radiation with an appropriate area. Besides, radiation becomes highly dense as a result of convergence at the rear end of the wave collecting unit 7, allowing accurate measurement of the temperature and dose of radiation with a measuring instrument.

Additionally, measurement results are indicated by a sound, allowing the user to easily measure radiation simply by paying attention to the sound without viewing the meter.

While, in the above embodiment, only the radiation temperature is shown on the meter and the temperature and dose of radiation are indicated by a sound, both temperature and dose of radiation may be shown on the meter alone. It is also conceivable to indicate the temperature level and the magnitude of dose of radiation with two different sounds.

Further, temperature detection and the filter are not absolutely necessary.

In the above embodiment, the dose of radiation is measured with the sensor 9, making it possible to enter the measurement as is into the computer for use as basic data for creating a human body distribution diagram.

On the other hand, the sensor 9 may detect the presence/absence of radiation alone, producing an alarming sound or lighting up a light for indication while radiation is being detected.

Fig. 6 illustrates a radiation detector having the cylindrical wave collecting unit 7, with a conical converging portion 13 provided between the filter 8 and the sensor 9 at the rear of the wave collecting unit 7.

In this embodiment, only radiation passing through the filter 8 is converged and measured.

### INDUSTRIAL APPLICABILITY

According to the method of the present invention, it is possible to collect human radiation in a pinpointed manner, obtaining the dose or intensity thereof as quantitative numeric data. This data is entered into a computer for display in a human body distribution diagram, allowing easy specification of affected parts and commanding an overall bird's-eye view of the whole body condition for holistic medical care.

According to the device of the present invention, it is possible to collect radiation emitted from a human body in a pinpointed manner and obtain accurate data through detection with a sensor without requiring experience. Only radiation of a specific wavelength is extracted by a filter, thus cutting out radiation other than that radiated by affected parts and electromagnetic waves other than those radiated by human body during measurement.

**Table 1**

| Confirmation of Pain Sensation by SRD Radiation Detector (Before Operation) | |
|---|---|
| Data confirming the order of areas of pain sensation indicated by SRD radiation detector from 305 subjects | |
| Subjects who answered "The order of all five grades is correct" | 253 |
| Subjects who answered "The order of four out of five grades is correct" | 31 |
| Subjects who answered "The order of three out of five grades is correct" | 21 |
| Subjects who answered "The order of two out of five grades is correct" | 0 |
| Subjects who answered "The order of one out of five grades is correct" | 0 |
| Subjects who answered "The order of all five grades is wrong" | 0 |

**Table 2**

| Confirmation of Pain Sensation by SRD Radiation Detector (After Operation) | |
|---|---|
| Data confirming the order of areas of pain sensation indicated by SRD radiation detector from 305 subjects | |
| Subjects who answered "The order of all five grades is correct" | 287 |
| Subjects who answered "The order of four out of five grades is correct" | 18 |
| Subjects who answered "The order of three out of five grades is correct" | 0 |
| Subjects who answered "The order of two out of five grades is correct" | 0 |
| Subjects who answered "The order of one out of five grades is correct" | 0 |
| Subjects who answered "The order of all five grades is wrong" | 0 |

## Claims

1. A method for specifying affected parts comprising the steps of:
detecting radiation emitted from a human body in a pinpointed manner by a radiation detector;
inputting a detected radiation dose or intensity to a computer; and
displaying the radiation dose or intensity for each area of the human body in a human body distribution diagram on a monitor screen.

2. The method for specifying affected parts according to claim 1, wherein the radiation dose or intensity is measured as the radiation detector is being gradually distanced from the human body, and wherein the maximum distance at which the radiation detector can detect radiation rays is used as a measurement.

3. The method for specifying affected parts according to claim 1 or 2, wherein the measurement is divided into a plurality of grades and displayed in a different color in each grade for display on the monitor screen.

4. A device for specifying affected parts comprising:
a radiation detector for detecting radiation emitted from a human body in a pinpointed manner; and
a distance measurement device for measuring the distance between the human body and the radiation detector.

5. The device for specifying affected parts according to claim 4, wherein the radiation detector comprises:
a wave collecting unit disposed on one side of a casing for collecting radiation waves emitted from the human body in a pinpointed manner;
a sensor disposed at the rear of the wave collecting unit for sensing radiation; and
a detection/display unit that remains active while the sensor senses radiation.

6. The device for specifying affected parts according to claim 5, wherein a filter is disposed, between the wave collecting unit and the sensor, that passes only radiation of a given wavelength.

7. The device for specifying affected parts according to claim 4, wherein the distance measurement device is a distance sensor that can be worn in the palm of the hand or a finger.

8. The affected part specifying device according to claim 4, wherein the distance measurement device is a distance sensor that is integrated with the radiation detector.
